# EUROPEAN PATENT SPECIFICATION

(11) **EP 4 525 788 B1**
(45) Date of publication and mention of the grant of the patent: **02.12.2026**
(21) Application number: 23731416.6
(22) Date of filing: 15.05.2023
(51) Int. Cl.: A61F 2/24

(54) **OUTER SKIRT FOR AN EXPANDABLE PROSTHETIC HEART VALVE**
ÄUSSERE SCHÜRZE FÜR EINE EXPANDIERBARE HERZKLAPPENPROTHESE
JUPE EXTERNE POUR UNE VALVE CARDIAQUE PROTHÉTIQUE EXTENSIBLE

(30) Priority: 16.05.2022 US 202263342269 P
(43) Date of publication of application: 26.03.2025
(73) Proprietor: Edwards Lifesciences Corporation, Irvine, CA 92614 (US)
(72) Inventor: LEVI, Tamir, S., 3079892 Caesarea (IL); SHERMAN, Elena, 3079892 Caesarea (IL)
(74) Representative: Eisenführ Speiser
(86) International application number: PCT/US2023/022213
(87) International publication number: WO 2023/224897

(56) References cited:
- US-A1- 2016 374 802
- US-A1- 2017 086 971
- US-A1- 2018 071 089
- US-A1- 2018 263 769
- US-A1- 2019 046 315
- US-A1- 2020 205 964

## Description

### CROSS-REFERENCE TO RELATED APPLICATIONS

This application claims the benefit of U.S. Provisional Patent Application No. 63/342,269, filed May 16, 2022.

### FIELD

The present disclosure relates to expandable prosthetic heart valves, including outer skirts for prosthetic heart valves.

### BACKGROUND

The human heart can suffer from various valvular diseases. These valvular diseases can result in significant malfunctioning of the heart and ultimately require repair of the native valve or replacement of the native valve with an artificial valve. There are a number of known repair devices (e.g., stents) and artificial valves, as well as a number of known methods of implanting these devices and valves in humans. Percutaneous and minimally-invasive surgical approaches are used in various procedures to deliver prosthetic medical devices to locations inside the body that are not readily accessible by surgery or where access without surgery is desirable. In one specific example, a prosthetic heart valve can be mounted in a crimped state on the distal end of a delivery apparatus and advanced through the patient's vasculature (e.g., through a femoral artery and the aorta) until the prosthetic valve reaches the implantation site in the heart. The prosthetic valve is then expanded to its functional size, for example, by inflating a balloon on which the prosthetic valve is mounted, actuating a mechanical actuator that applies an expansion force to the prosthetic valve, or by deploying the prosthetic valve from a sheath of the delivery apparatus so that the prosthetic valve can self-expand to its functional size.

Most expandable, prosthetic heart valves comprise a radially expandable and compressible cylindrical metal frame or stent and prosthetic leaflets mounted inside the frame. Commissures can be formed by connecting pairs of commissure tabs of adjacent leaflets to each other and to commissure windows formed in the frame. Prosthetic heart valves can also include an outer covering or skirt disposed around an outer surface of the frame. **In** some examples, the prosthetic heart valve can be radially compressed (crimped) onto the delivery apparatus and then advanced through a delivery sheath inserted into a patient's vessel using the delivery apparatus. The delivery sheath can provide a path to the target implantation for the delivery apparatus to navigate through. However, in some instances, push forces experienced by a user advancing the delivery apparatus through the delivery sheath can be greater than desired. Accordingly, improvements to the prosthetic heart valve that decrease these push forces is desirable.

US 2017/0086971 A1 relates to a prosthetic heart valve for replacing a native valve which includes a collapsible and expandable stent extending between a proximal end and a distal end. The stent includes an annulus section adjacent the proximal end and having a first diameter, a plurality of first struts forming cells, and a plurality of second struts connected to the annulus section and forming a plurality of deflecting cells expandable to define a second diameter larger than the first diameter. A valve assembly is disposed within the stent and a cuff is coupled to the stent and covers the plurality of deflecting cells.

US 2018/0071089 A1 relates to a prosthetic heart valve which includes a collapsible and expandable stent with a valve assembly disposed therein. A first cuff is positioned on the lumenal or ablumenal surface of the stent. A second cuff is positioned radially outward of the stent and the first cuff. The second cuff may include apertures that allow blood to pass through the second cuff into the spaces between the first and second cuffs. The second cuff may include a proximal edge with a plurality of notches that may be closed to create puckered areas in the second cuff to facilitate the movement of blood in the spaces between the first and second cuffs. The stent may include struts adjacent the second cuff that bow radially inwardly to create additional space for blood to flow in the spaces between the first and second cuffs.

US 2020/0205964 A1 relates to an implantable prosthetic valve that is radially collapsible to a collapsed configuration and radially expandable to an expanded configuration and includes an annular frame including a plurality of angled strut members, and a skirt member secured to the frame. The skirt member includes a plurality of extension portions wrapped around at least one strut member adjacent the extension portions. The prosthetic valve further includes a cord member threaded through at least a portion of the plurality of extension portions to secure the skirt member to the at least one strut member.

US 2019/0046315 A1 relates to an implantable prosthetic valve assembly which comprises a frame assembly including a plurality of struts and a prosthetic valve secured inside the frame assembly by sutures. The sutures are inward of the outermost surface of an outer periphery of the frame assembly. In certain configurations the struts contain recesses in which sutures can be disposed. In other configurations, the struts can have holes through them which the sutures pass through. The implantable prosthetic valve assembly can also be collapsed within a catheter such that an outermost surface of a periphery of the frame assembly touches the catheter but the sutures are spaced apart from the catheter or are flush with the outer surface of the frame.

US 2016/374802 A1 relates to a prosthetic heart valve with an outer skirt comprising projections and with commissure portions arranged on the exterior of the frame.

### SUMMARY

The claimed invention as defined in independent claim 1 relates to a prosthetic heart valve. Some preferred configurations of the claimed prosthetic heart valve are defined in dependent claims 2 to 10. The claimed invention also relates to a prosthetic heart valve as defined in independent claim 11, some preferred configurations of which are defined in dependent claims 12 to 15. Also described herein are related aspects, examples, embodiments and arrangements useful for understanding the claimed invention, and which do not necessarily constitute embodiments of the claimed invention. The subject-matter for which protection is sought is defined by the claims.

Described herein are prosthetic heart valves, delivery apparatuses, and methods for implanting prosthetic heart valves. In particular, described herein are examples of outer coverings or skirts for prosthetic heart valves, and methods of making and using such outer skirts. Prosthetic heart valves can include a frame and a leaflet assembly arranged on an inner surface of the frame. Commissures can be formed by connecting pairs of commissure tabs of adjacent leaflets to each other and to commissure windows formed in the frame. The prosthetic heart valve can include an outer skirt arranged around a circumference of the frame and on an outer surface of the frame. The outer skirt can include one or more extension portions extending axially outward from an edge portion of the outer skirt. Each extension portion can be configured to cover at least a portion of a corresponding commissure, thereby reducing push forces when advancing a radially compressed prosthetic heart valve mounted on a delivery apparatus through a delivery sheath extending through a vessel of a patient toward a target implantation site. As such, the skirts and prosthetic heart valves disclosed herein can, among other things, overcome one or more of the deficiencies of typical prosthetic heart valves and their delivery apparatuses.

A prosthetic heart valve can comprise a frame and a valvular structure coupled to the frame. In addition to these components, a prosthetic heart valve can further comprise one or more of the components disclosed herein.

In some examples, the prosthetic heart valve can comprise a sealing member configured to reduce paravalvular leakage.

In some examples, the sealing member can be an outer skirt disposed around an outer surface of the frame, the outer skirt comprising a first edge portion, a second edge portion arranged at an intermediate portion of the frame that is disposed between a first end and a second end of the frame, and one or more extension portions extending axially from the second edge portion.

In some examples, the frame can comprise a plurality of interconnected struts comprising a plurality of axially extending window struts forming a plurality of commissure windows spaced apart around the frame, and the one or more extension portions can extend over at least a portion of a respective commissure window of the plurality of commissure windows.

In some examples, the valvular structure can comprise a plurality of leaflets arranged within an interior of the frame and secured to one another at adjacent ends to form commissures, where each commissure is connected to a respective commissure feature of the frame. The one or more extension portions can extend axially from the second edge portion, each extension portion covering at least a portion of a corresponding commissure on an exterior of the frame.

In some examples, the one or more extension portions comprise a plurality of extension portions.

In some examples, the outer skirt can comprise a first stitch line and a second stitch line extending circumferentially along an inflow portion of the outer skirt that includes the first edge portion. The first stitch line and the second stitch line are spaced axially apart from one another, and each comprise a plurality of in-and-out stitches.

In some examples, a prosthetic heart valve comprises an annular frame comprising a plurality of interconnected struts, the plurality of interconnected struts comprising a plurality of axially extending window struts forming a plurality of commissure windows spaced apart around the frame. The prosthetic heart valve further comprises an outer skirt disposed around an outer surface of the frame, the outer skirt comprising a first edge portion, a second edge portion arranged at an intermediate portion of the frame that is disposed between a first end and a second end of the frame, and one or more extension portions extending axially from the second edge portion and over at least a portion of a respective commissure window of the plurality of commissure windows.

In some examples, a prosthetic heart valve comprises an annular frame comprising a plurality of commissure features and a plurality of leaflets arranged within an interior of the frame and secured to one another at adjacent ends to form commissures, where each commissure is connected to a respective commissure feature of the frame. The prosthetic heart valve further comprises an outer skirt disposed around an outer surface of the frame, the outer skirt comprising a first edge portion, a second edge portion secured to an intermediate portion of the frame disposed between a first end and a second end of the frame, and a plurality of extension portions extending axially from the second edge portion, each extension portion covering a at least a portion of a corresponding commissure on an exterior of the frame.

In some examples, a prosthetic heart valve comprises an annular frame comprising a plurality of interconnected struts and having an inflow end and an outflow end, and an outer skirt disposed around an outer surface of the frame. The outer skirt comprises an inflow edge portion disposed at the inflow end, an outflow edge portion disposed at an intermediate portion of the frame, a plurality of extension portions extending axially from the outflow edge portion and spaced circumferentially apart from one another, where each extension portion extends toward the outflow end of the frame, and a first stitch line and a second stitch line extending circumferentially along an inflow portion of the outer skirt that includes the inflow edge portion. The first stitch line and the second stitch line are spaced axially apart from one another, and each comprise a plurality of in-and-out stitches.

In some examples, a prosthetic heart valve comprises an annular frame comprising a plurality of interconnected struts defining a plurality of rows of cells arranged between an inflow end and an outflow end of the frame, the plurality of interconnected struts comprising a plurality of outflow struts defining the outflow end and a plurality of inflow struts defining the inflow end. The prosthetic heart valve further comprises an outer skirt disposed around an outer surface of the frame, the outer skirt comprising: an outflow edge portion disposed at an intermediate portion of the frame, and an inflow portion comprising an inflow edge portion, a first stitch line extending circumferentially along the inflow portion adjacent to the inflow edge portion, and a second stitch line extending circumferentially along the inflow portion adjacent to but spaced axially away from the first stitch line. The inflow portion is wrapped around the plurality of inflow struts such that the first stitch line is disposed on an interior of the frame and the second stitch line is disposed on an exterior of the frame.

In some examples, a prosthetic heart valve comprises one or more of the components recited in Examples 1-55 below.

The various innovations of this disclosure can be used in combination or separately. This summary is provided to introduce a selection of concepts in a simplified form that are further described below in the detailed description. The foregoing and other objects, features, and advantages of the disclosure will become more apparent from the following detailed description, claims, and accompanying figures.

### BRIEF DESCRIPTION OF THE DRAWINGS

FIG. 1 is a side view of a prosthetic heart valve, according to one example.
FIG. 2 is a side view of a frame of the prosthetic heart valve of FIG. 1.
FIG. 3 is a side view of a portion of the frame of FIG. 2, showing the portion of the frame in a straightened (non-annular) state.
FIG. 4 is a side view of an exemplary delivery apparatus configured to deliver and implant a radially expandable prosthetic heart valve at an implantation site.
FIG. 5 is a side view of an outer skirt for a prosthetic heart valve, according to an example, the outer skirt shown in a flattened configuration and including extension portions extending outward, in an axial direction, from an edge portion of the outer skirt.
FIG. 6 is a detail view of a portion of the outer skirt of FIG. 5 showing a plurality of stitching lines across the outer skirt.
FIG. 7 is a side view of an exemplary prosthetic heart valve with the outer skirt of FIG. 5 disposed around and secured to an outer surface of a frame of the prosthetic heart valve.
FIG. 8 is a detail view of an inflow end portion of the prosthetic heart valve of FIG. 7 showing an inflow portion of the outer skirt wrapped around and secured to inflow struts of the frame.
FIG. 9 is a first cross-sectional view of the prosthetic heart valve of FIG. 7 showing an extension portion of the outer skirt covering a portion of a commissure of the prosthetic heart valve.
FIG. 10 is a second cross-sectional view of the prosthetic heart valve of FIG. 7 showing an inflow portion of the outer skirt wrapped around an inflow strut of the frame.
FIG. 11 is a detail view of a portion of FIG. 10 showing the inflow portion of the outer skirt secured to the inflow strut using first and second stitch lines of the outer skirt.

### DETAILED DESCRIPTION

### General Considerations

For purposes of this description, certain aspects, advantages, and novel features of examples of this disclosure are described herein. The disclosed methods, apparatus, and systems should not be construed as being limiting in any way. Instead, the present disclosure is directed toward all novel and nonobvious features and aspects of the various disclosed examples, alone and in various combinations and sub-combinations with one another. The methods, apparatus, and systems are not limited to any specific aspect or feature or combination thereof, nor do the disclosed examples require that any one or more specific advantages be present or problems be solved.

Although the operations of some of the disclosed examples are described in a particular, sequential order for convenient presentation, it should be understood that this manner of description encompasses rearrangement, unless a particular ordering is required by specific language set forth below. For example, operations described sequentially may in some cases be rearranged or performed concurrently. Moreover, for the sake of simplicity, the attached figures may not show the various ways in which the disclosed methods can be used in conjunction with other methods. Additionally, the description sometimes uses terms like "provide" or "achieve" to describe the disclosed methods. These terms are high-level abstractions of the actual operations that are performed. The actual operations that correspond to these terms may vary depending on the particular implementation and are readily discernible by one of ordinary skill in the art.

As used in this application and in the claims, the singular forms "a," "an," and "the" include the plural forms unless the context clearly dictates otherwise. Additionally, the term "includes" means "comprises." Further, the term "coupled" generally means physically, mechanically, chemically, magnetically, and/or electrically coupled or linked and does not exclude the presence of intermediate elements between the coupled or associated items absent specific contrary language.

As used herein, the term "proximal" refers to a position, direction, or portion of a device that is closer to the user and further away from the implantation site. As used herein, the term "distal" refers to a position, direction, or portion of a device that is further away from the user and closer to the implantation site. Thus, for example, proximal motion of a device is motion of the device away from the implantation site and toward the user (e.g., out of the patient's body), while distal motion of the device is motion of the device away from the user and toward the implantation site (e.g., into the patient's body). The terms "longitudinal" and "axial" refer to an axis extending in the proximal and distal directions, unless otherwise expressly defined.

### Overview of the Disclosed Technology

As introduced above, prosthetic heart valves can include leaflets that are secured to one another at their adjacent sides (e.g., commissure tabs) to form commissures which are secured to a frame of the prosthetic heart valve. In some examples, the commissures can extend through commissure windows in the frame of the prosthetic heart valve and then be secured to the frame struts forming the commissures windows. Thus, a portion of the commissures can extend radially outward and away from an outer surface of the frame. This can form step-like or protruding features on an exterior of the prosthetic heart valve. For delivery to an implantation site, the prosthetic heart valve is radially compressed around a distal end portion of a delivery apparatus, which is then navigated through a delivery sheath that has been inserted into a vessel of a patient and extends toward the implantation site. As the delivery apparatus is moved through the delivery sheath, the protruding commissures may contact the inner wall of the delivery sheath, thereby causing the commissures to fold backward and/or press against the inner wall of the delivery sheath as the radially compressed prosthetic heart valve is pushed through the delivery sheath. As a result, a push force felt by a user advancing the delivery apparatus through the delivery sheath can increase.

Described herein are various examples of outer skirts for prosthetic heart valves that are disposed around an outer surface of the prosthetic heart valve and that are configured to form a seal against native tissue upon implantation of the prosthetic heart valve, thereby reducing paravalvular leakage (PVL) past the prosthetic heart valve when expanded against the native anatomy. The outer skirts described herein includes vertical (or axial) extensions at an outflow end portion of the outer skirt, the vertical extensions covering at least an inflow end portion of the commissures, thereby providing a tapered and smoother surface that can facilitate easier advancement of the prosthetic heart valve through the delivery sheath, and thus reduced push forces felt by a user.

Prosthetic valves disclosed herein can be radially compressible and expandable between a radially compressed state and a radially expanded state. Thus, the prosthetic valves can be crimped on or retained by an implant delivery apparatus in the radially compressed state while being advanced through a patient's vasculature on the delivery apparatus. The prosthetic valve can be expanded to the radially expanded state once the prosthetic valve reaches the implantation site. It is understood that the prosthetic valves disclosed herein may be used with a variety of implant delivery apparatuses and can be implanted via various delivery procedures, examples of which will be discussed in more detail later.

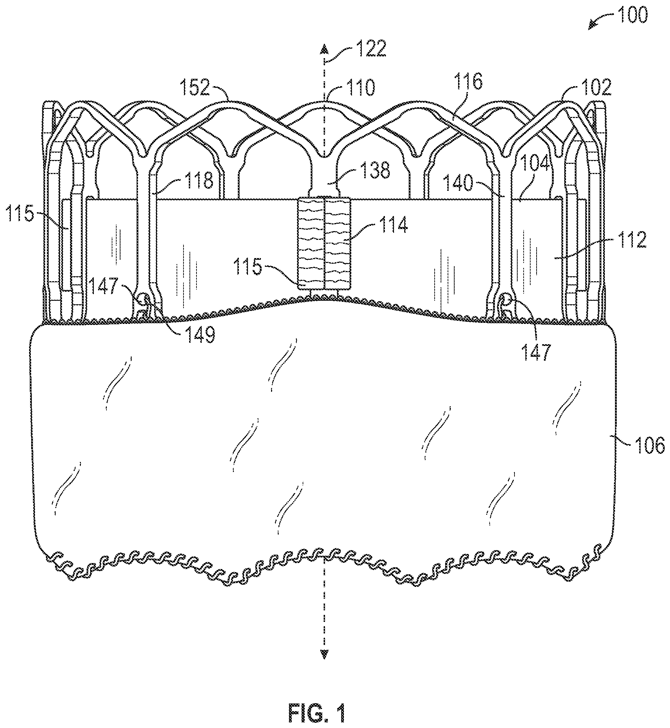

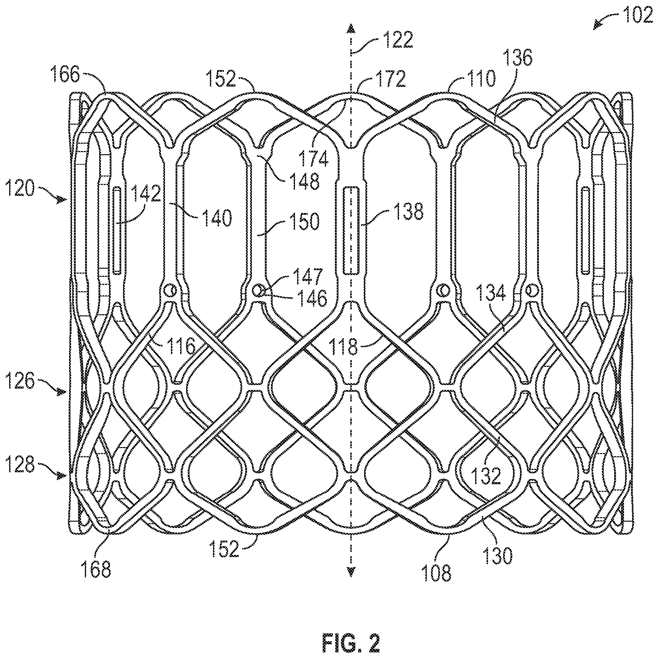

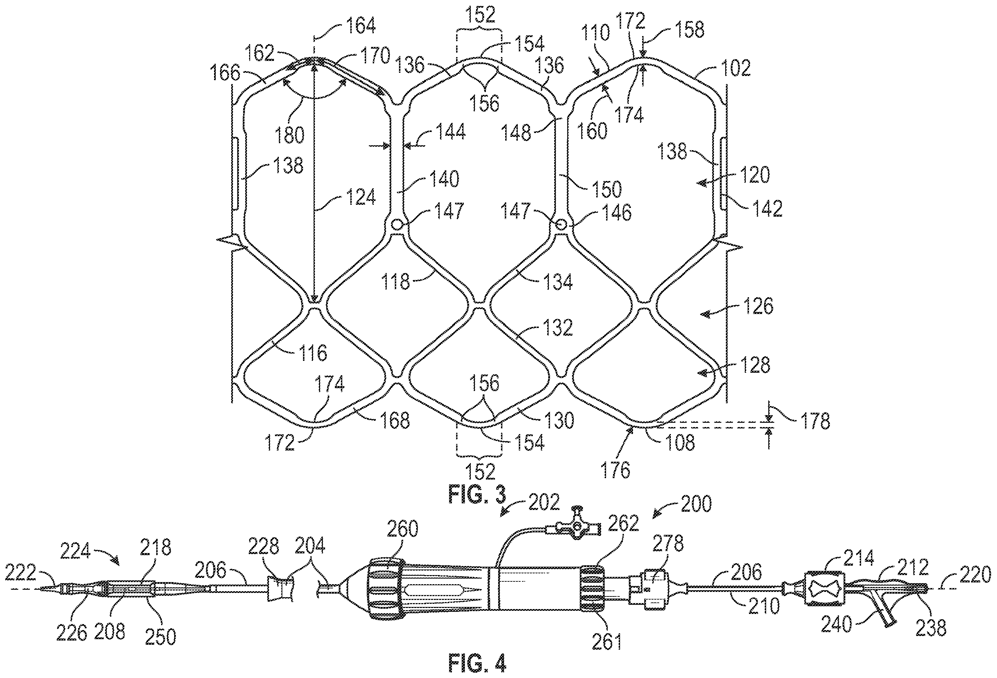

FIG. 1 illustrates an exemplary prosthetic device (e.g., prosthetic heart valve) comprising a frame, leaflets secured on an inside of the frame, and an outer skirt disposed around an outer surface of the frame. In some examples, the frame can comprise a plurality of interconnected and angled struts and apex regions that extend and/or curve between the angled struts at an inflow end and outflow end of the frame, as shown in FIGS. 2 and 3. The prosthetic device can be advanced through a patient's vasculature, such as to a native heart valve, by a delivery apparatus, such as the exemplary delivery apparatus shown in FIG. 4.

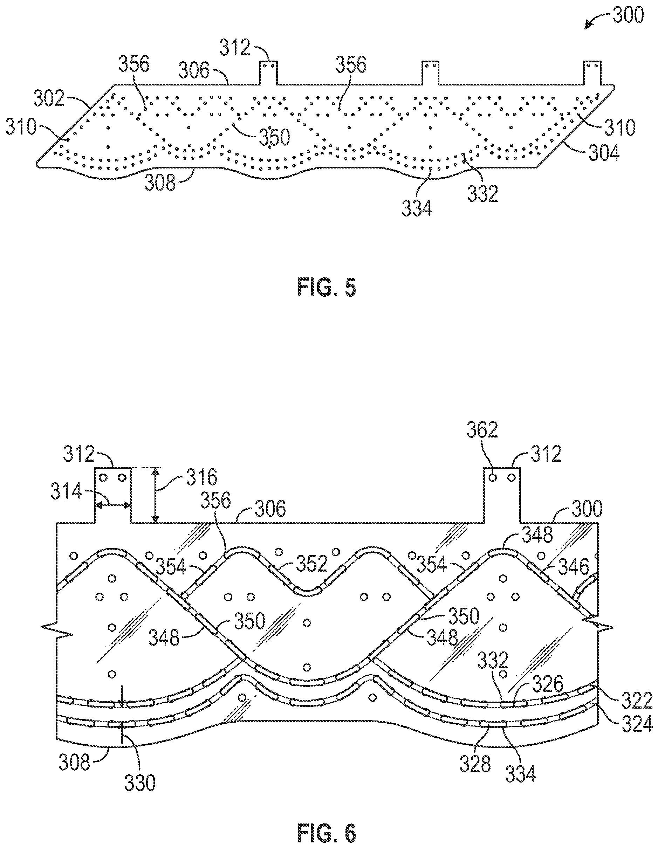

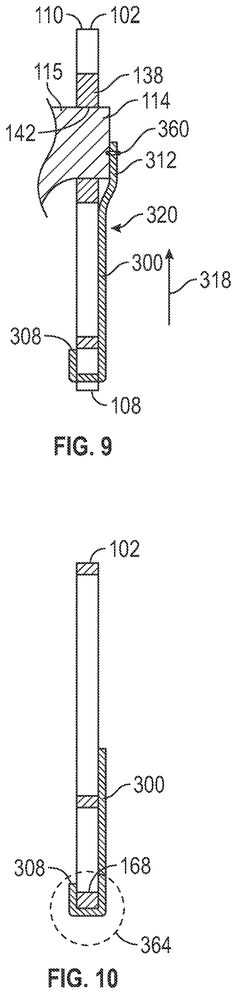

In some examples, an outer skirt for a prosthetic heart valve, such as the outer skirt depicted in FIGS. 5-7, is configured with axially extending extension potions that extend outward from an outflow edge portion of the outer skirt. The extension portions are configured to extend over a portion of the commissures protruding radially outward from commissure windows of the frame (as shown in FIG. 9). The extension portions can provide a tapered and/or smoother (or lower friction) surface covering an inflow portion of the commissures, thereby decreasing push forces felt by a user as the radially compressed prosthetic heart valve is advanced through a delivery sheath via a delivery apparatus.

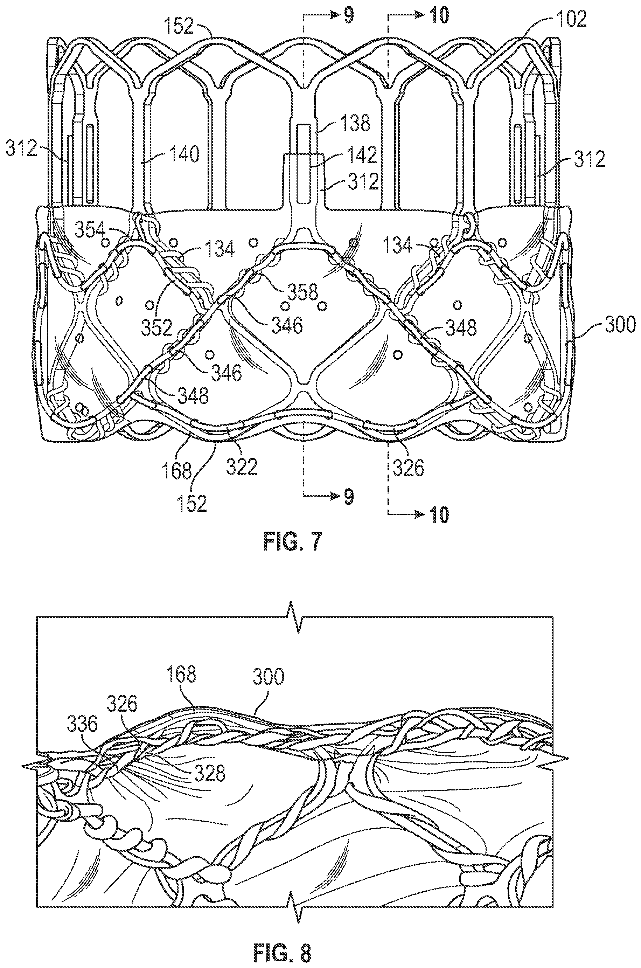

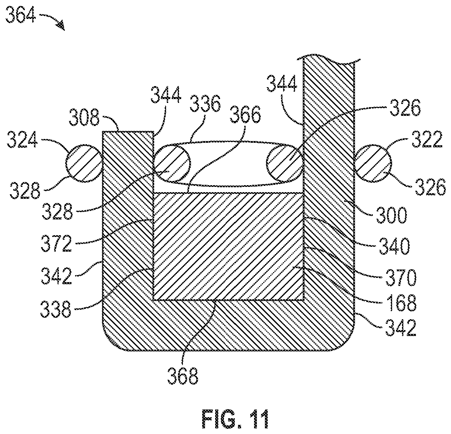

In some examples, as depicted in FIG 6, the outer skirt further includes two spaced apart stitch lines extending circumferentially along an inflow portion of the outer skirt and including a plurality of in-and-out stitches. The inflow portion of the outer skirt is wrapped around the inflow end of the frame, as shown in FIGS. 7-11, such that the first stitch line is disposed on an exterior of the frame and the second stitch line is disposed on an interior of the frame (as shown in FIG. 11). The stitches of the first and second stitches are then secured together, thereby securing the outer skirt over and around the inflow end of the frame. As a result, inflow struts defining the inflow end of the frame can be covered, thereby reducing potential abrasion of the inflow struts against the delivery sheath and/or further reducing push forces as the prosthetic heart valve is advanced through the delivery sheath.

### Examples of the Disclosed Technology

FIG. 1 shows a prosthetic heart valve 100 (prosthetic valve), according to one example. Any of the prosthetic valves disclosed herein are adapted to be implanted in the native aortic annulus, although in other examples they can be adapted to be implanted in the other native annuluses of the heart (the pulmonary, mitral, and tricuspid valves). The disclosed prosthetic valves also can be implanted within vessels communicating with the heart, including a pulmonary artery (for replacing the function of a diseased pulmonary valve, or the superior vena cava or the inferior vena cava (for replacing the function of a diseased tricuspid valve) or various other veins, arteries and vessels of a patient. The disclosed prosthetic valves also can be implanted within a previously implanted prosthetic valve (which can be a prosthetic surgical valve or a prosthetic transcatheter heart valve) in a valve-in-valve procedure.

In some examples, the disclosed prosthetic valves can be implanted within a docking or anchoring device that is implanted within a native heart valve or a vessel. For example, in one example, the disclosed prosthetic valves can be implanted within a docking device implanted within the pulmonary artery for replacing the function of a diseased pulmonary valve, such as disclosed in U.S. Publication No. 2017/0231756. In another example, the disclosed prosthetic valves can be implanted within a docking device implanted within or at the native mitral valve, such as disclosed in PCT Publication No. WO2020/247907. In another example, the disclosed prosthetic valves can be implanted within a docking device implanted within the superior or inferior vena cava for replacing the function of a diseased tricuspid valve, such as disclosed in U.S. Publication No. 2019/0000615.

The prosthetic heart valve 100 can include a stent or frame 102, a valvular structure 104, and a perivalvular outer sealing member or outer skirt 106. The prosthetic heart valve 100 (and the frame 102) can have an inflow end 108 and an outflow end 110. The valvular structure 104 can be disposed on an interior of the frame 102 while the outer skirt 106 is disposed around an outer surface of the frame 102.

The valvular structure 104 can comprise a plurality of leaflets 112 (e.g., three leaflets, as shown in FIG. 1), collectively forming a leaflet structure, which can be arranged to collapse in a tricuspid arrangement. The leaflets 112 can be secured to one another at their adjacent sides (e.g., commissure tabs) to form commissures 114 of the valvular structure 104. For example, each leaflet 112 can comprise opposing commissure tabs disposed on opposite sides of the leaflet 112 and a cusp edge portion extending between the opposing commissure tabs. The cusp edge portion of the leaflets 112 can have an undulating, curved scalloped shape, and can be secured directly to the frame 102 (e.g., by sutures). However, in alternate examples, the cusp edge portion of the leaflets 112 can be secured to an inner skirt which is then secured to the frame 102. In some examples, the leaflets 112 can be formed of pericardial tissue (e.g., bovine pericardial tissue), biocompatible synthetic materials, or various other suitable natural or synthetic materials as known in the art and described in U.S. Patent No. 6,730,118.

In some examples, the outer skirt 106 can be an annular skirt. In some instances, the outer skirt 106 can comprise one or more skirt portions that are connected together and/or individually connected to the frame 102. The outer skirt 106 can comprise a fabric or polymeric material, such as ePTFE, PTFE, PET, TPU, UHMWPE, PEEK, PE, etc. In some instances, instead of having a relatively straight upper edge portion, as shown in FIG. 1, the outer skirt 106 can have an undulating upper edge portion that extends along and is secured to the angled struts 134. Examples of such outer skirts, as well as various other outer skirts, that can be used with the frame 102 can be found in the provisional patent application under Edwards attorney docket No. 12131US01.

The frame 102 can be radially compressible and expandable between a radially compressed configuration and a radially expanded configuration (the expanded configuration is shown in FIG. 1). The frame 102 is shown alone in FIG. 2 and a portion of the frame 102 in a straightened (non-annular) configuration is shown in FIG. 3.

The frame 102 can be made of any of various suitable plastically-expandable materials (e.g., stainless steel, etc.) or self-expanding materials (e.g., Nitinol). When constructed of a plastically-expandable material, the frame 102 (and thus the valve 100) can be crimped to a radially compressed state on a delivery catheter and then expanded inside a patient by an inflatable balloon or equivalent expansion mechanism. When constructed of a self-expandable material, the frame 102 (and thus the valve 100) can be crimped to a radially compressed state and restrained in the compressed state by insertion into a sheath or equivalent mechanism of a delivery catheter. Once inside the body, the valve can be advanced from the delivery sheath, which allows the valve to expand to its functional size.

Suitable plastically-expandable materials that can be used to form the frame 102 include, metal alloys, polymers, or combinations thereof. Example metal alloys can comprise one or more of the following: nickel, cobalt, chromium, molybdenum, titanium, or other biocompatible metal. In some examples, the frame 102 can comprise stainless steel. In some examples, the frame 102 can comprise cobalt-chromium. In some examples, the frame 102 can comprise nickel-cobalt-chromium. In some examples, the frame 102 comprises a nickel-cobalt-chromium-molybdenum alloy, such as MP35N^{™} (tradename of SPS Technologies), which is equivalent to UNS R30035 (covered by ASTM F562-02). MP35N^{™}/UNS R30035 comprises 35% nickel, 35% cobalt, 20% chromium, and 10% molybdenum, by weight.

As shown in FIGS. 2 and 3, the frame 102 can comprise a plurality of interconnected struts 116 which form multiple rows of open cells 118 between the outflow end 110 and the inflow end 108 of the frame 102. In some examples, as shown in FIGS. 2 and 3, the frame 102 can comprise three rows of cells 118 with a first (upper in the orientation shown in FIGS. 2 and 3) row of cells 120 disposed at the outflow end 110. The first row of cells 120 comprises cells 118 that are elongated in an axial direction (relative to a central longitudinal axis 122 of the frame 102), as compared to cells 118 in the remaining rows of cells. For example, the cells 118 of the first row of cells 120 can have a longer axial length 124 (FIG. 3) than cells 118 in the remaining rows of cells, which can include a second row of cells 126 and a third row of cells 128, the third row of cells 128 disposed at the inflow end 108 and the second row of cells 126 disposed between the first row of cells 120 and the third row of cells 128.

In some examples, as shown in FIG. 2, each row of cells comprises nine cells 118. Thus, in such examples, the frame 102 can be referred to as a nine-cell frame.

In alternate examples, the frame 102 can comprise more than three rows of cells (e.g., four or five) and/or more or less than nine cells per row. In some examples, the cells 118 in the first row of cells 120 may not be elongated compared to cells 118 in the remaining rows of cells of the frame 102 (the second row of cells 126 and the third row of cells 128).

The interconnected struts 116 can include a plurality of angled struts 130, 132, 134, and 136 arranged in a plurality of rows of circumferentially extending rows of angled struts, with the rows being arrayed along the length of the frame 102 between the outflow end 110 and the inflow end 108. For example, the frame 102 can comprise a first row of angled struts 130 arranged end-to-end and extending circumferentially at the inflow end 108 of the frame; a second row of circumferentially extending, angled struts 132; a third row of circumferentially extending, angled struts 134; and a fourth row of circumferentially extending, angled struts 136 at the outflow end 110 of the frame 102. The fourth row of angled struts 136 can be connected to the third row of angled struts 134 by a plurality of axially extending window struts 138 (or window strut portions) and a plurality of axial (e.g., axially extending) struts 140. The axially extending window struts 138 (which can also be referred to as axial struts that include a commissure window) define commissure windows (e.g., open windows) 142 that are spaced apart from one another around the frame 102, in a circumferential direction, and which are adapted to receive a pair of commissure tabs of a pair of adjacent leaflets 112 arranged into a commissure (e.g., commissure 114 shown in FIG. 1). In some examples, the commissure windows 142 and/or the axially extending window struts 138 defining the commissure windows 142 can be referred to herein as commissure features or commissure supports, each commissure feature or support configured to receive and/or be secured to a pair of commissure tabs of a pair of adjacent leaflets.

One or more (e.g., two, as shown in FIGS. 2 and 3) axial struts 140 can be positioned between, in the circumferential direction, two commissure windows 142 formed by the window struts 138. Since the frame 102 can include fewer cells per row (e.g., nine) and fewer axial struts 140 between each commissure window 142, as compared to some more traditional prosthetic heart valves, each cell 118 can have an increased width (in the circumferential direction), thereby providing a larger opening for blood flow and/or coronary access.

Each axial strut 140 and each window strut 138 extends from a location defined by the convergence of the lower ends (e.g., ends arranged inward of and farthest away from the outflow end 110) of two angled struts 136 (which can also be referred to as an upper strut junction or upper elongated strut junction) to another location defined by the convergence of the upper ends (e.g., ends arranged closer to the outflow end 110) of two angled struts 134 (which can also be referred to as a lower strut junction or lower elongate strut junction). Each axial strut 140 and each window strut 138 forms an axial side of two adjacent cells of the first row of cells 120.

In some examples, as shown in FIG. 3, each axial strut 140 can have a width 144 (FIG. 3) that is larger than a width of the angled struts 130, 132, 134, and 136. As used herein, a "width" of a strut is measured between opposing locations on opposing surfaces of a strut that extend between the radially facing inner and outer surfaces of the strut (relative to the central longitudinal axis 122 of the frame 102). A "thickness" of a strut is measured between opposing locations on the radially facing inner and outer surfaces of a strut and is perpendicular to the width of the strut. In some examples, the width 144 of the axial struts 140 is 50-200%, 75-150%, or at least 100% larger than (e.g., double) the width of the angled struts of the frame 102.

By providing the axial struts 140 with the width 144 that is greater than the width of other, angled struts of the frame 102, a larger contact area is provided for when the leaflets 112 contact the wider axial struts 140 during systole, thereby distributing the stress and reducing the extent to which the leaflets 112 may fold over the axial struts 140, radially outward through the cells 118. As a result, a long-term durability of the leaflets 112 can be increased.

Since the cells 118 of the frame 102 can have a relatively large width compared to alternate prosthetic valves that have more than nine cells per row (as introduced above), the wider axial struts 140 can be more easily incorporated into the frame 102, without sacrificing open space for blood flow and/or coronary access.

Commissure tabs 115 of adjacent leaflets 112 can be secured together to form commissures 114 (FIG. 1). Each commissure 114 of the prosthetic heart valve 100 comprises two commissure tabs 115 paired together, one from each of two adjacent leaflets 112, and extending through a commissure window 142 of the frame 102. Each commissure 114 can be secured to the window struts 138 forming the commissure window 142.

The cusp edge portion (e.g., scallop edge) of each leaflet 112 can be secured to the frame 102 via one or more fasteners (e.g., sutures). In some examples, the cusp edge portion of each leaflet 112 can be secured directly to the struts of the frame 102 (e.g., angled struts 130, 132, and 134). For example, the cusp edge portions of the leaflets 112 can be sutured to the angled struts 130, 132, and 134 that generally follow the contour of the cusp edge portions of the leaflets 112.

In some examples, the cusp edge portion of the leaflets 112 can be secured to an inner skirt and the inner skirt can then be secured directly to the frame 102.

Various methods for securing the leaflets 112 to a frame, such as the frame 102, are disclosed in U.S. provisional patent applications 63/278,922, filed November 12, 2021, and 63/300,302, filed January 18, 2022.

As shown in FIGS. 2 and 3, in some examples, one or more of or each of the axial struts 140 can comprise an inflow end portion 146 (e.g., an end portion that is closest to the inflow end 108) and an outflow end portion 148 that are widened relative to a middle portion 150 of the axial strut 140 (which can be defined by the width 144). In some instances, the inflow end portion 146 of the axial strut 140 can comprise an aperture 147. The apertures 147 can be configured to receive fasteners (e.g., sutures) for attaching soft components of the prosthetic heart valve 100 to the frame 102. For example, in some instances, the outer skirt 106 can be positioned around the outer surface of the frame 102 and an upper or outflow edge portion of the outer skirt 106 can be secured to the apertures 147 by fasteners 149 (e.g., sutures), as shown in FIG. 1.

The frame 102 can further comprise a plurality of apex regions 152 formed at the inflow end 108 and the outflow end 110, each apex region 152 extending and forming a junction between two angled struts 130 at the inflow end 108 or two angled struts 136 at the outflow end 110. As such, the apex regions 152 are spaced apart from one another, in a circumferential direction at the inflow end 108 and the outflow end 110.

Each apex region 152 can comprise an apex 154 (the highest or most outward extending, in an axial direction, point) and two thinned (or narrowed) strut portions 156, one thinned strut portion 156 extending from either side of the apex 154 to a corresponding, wider, angled strut 136 (at the outflow end 110) or angled strut 130 (at the inflow end 108) (FIG. 3). In this way, each of the apex regions 152 at the outflow end 110 can form a narrowed transition region between and relative to the two angled struts 136 extending from the corresponding apex region 152 and each of the apex regions 152 at the inflow end 108 can form a narrowed transition region between and relative to the two angled struts 130 extending from the corresponding apex region 152.

The thinned strut portions 156 of the apex regions 152 can have a width 158 that is smaller than a width 160 of the angled struts 130 or 136 (FIG. 3). In some examples, the width 158 can be a uniform width (e.g., along an entire length of the strut portion 156). In some examples, the width 158 of the thinned strut portions 156 can be from about 0.06 - 0.15 mm smaller than the width 160 of the angled struts 130 and/or 136.

The thinned strut portions 156 of the apex regions 152 can have a first length 162 (FIG. 3). In some examples, the first length 162 is in a range of 0.8-1.4 mm, 0.9-1.2 mm, 0.95-1.05 mm, or about 1.0 mm (e.g., ±0.03 mm). In alternate examples, the first length 162 is in a range of 0.3-0.7 mm, 0.4-0.6 mm, 0.45-0.55 mm, or about 0.5 mm (e.g., ±0.03 mm).

Thus, each outflow apex region 152 can include two thinned strut portions 156 having the first length 162, each extending from the apex 154, outward relative to a central longitudinal axis 164 of the cells 118. Thus, a total length of the apex region 152 can be two times the first length 162.

Each apex region 152 and two corresponding angled struts 136 at the outflow end 110 can form an outflow strut 166 and each apex region 152 and two corresponding angled struts 130 at the inflow end 108 can form an inflow strut 168.

Each outflow strut 166 and inflow strut 168 can have a length that includes an apex region 152 and the two angled struts 136 or 130 (or strut portions), respectively, on either side of the apex region 152. One half the total length of each outflow strut 166 and inflow strut 168 is shown in FIG. 3 as length 170, which extends from an end of one angled strut 136 or 130 to the central longitudinal axis 164. Thus, the length of each outflow strut 166 and inflow strut 168 is two times length 170. In some examples, the length 170 for half of each inflow strut 168 can be different than the length 170 for half of each outflow strut 166.

In some instances, the length of each thinned strut portion 156 can be at least 25% of the length 170 of the corresponding half outflow strut 166 or inflow strut 168. Said another way, the length of each apex region 152 (a total length being two times the first length 162) can be at least 25% of the total length (two times length 170) of the outflow strut 166 or inflow strut 168. In some examples, the length of each apex region 152 can be more than 25% of the total length of the corresponding outflow strut 166 or inflow strut 168, such as 25-35%.

In some examples, each apex region 152 can comprise a curved, axially facing outer surface 172 and an arcuate or curved, axially facing inner depression 174 which forms the thinned strut portions 156. For example, the curved inner depression 174 can depress toward the curved outer surface 172 from an inner surface of the angled strut portions 156, thereby forming the smaller width thinned strut portions 156. Thus, the curved inner depressions 174 can be formed on a cell side of the apex region 152 (e.g., as opposed to the outside of the apex region 152).

In some examples, the curved outer surface 172 of each apex region 152 can form a single, continuous curve from one angled strut portion 156 on a first side of the apex region 152 to another angled strut portion 156 on an opposite, second side of the apex region 152.

Each apex region 152 can have a radius of curvature 176, along the curved outer surface 172 (e.g., in some instances, along an entirety or an entire length of the curved outer surface 172) (FIG. 3). In some instances, the radius of curvature 176 at the apex 154 and/or along the entire curved outer surface 172 of the apex region 152 can be greater than 1 mm. In some instances, the radius of curvature 176 can be in a range of 1-20 mm, 3-16 mm, or 8-14 mm. In some instances, the radius of curvature 176 can be greater than 10 mm. The radius of curvature 176 can be dependent on (and thus change due to changes in) the width 158 (e.g., the amount of reduction in width from the angled struts 130 or 136) and the first length 162 of the thinned strut portions 156.

Further, a height (an axial height) 178 of the apex regions 152, which can be defined in the axial direction from an outer surface of the two angled struts 130 or 136 to the curved outer surface 172 of the apex region 152 at the apex 414, can be the width 158 of the thinned strut portions 156 (FIG. 3). In this way, the height 178 of the apex regions 152 can be relatively small and not add much to the overall axial height of the radially expanded frame 102. Thus, the leaflets 112 secured to the frame 102 (FIG. 1) can be disposed close to the inflow end 108, thereby leaving a lar If ger open space at the outflow end 110 of the frame 102 that is not blocked by the leaflets 112.

In some examples, each of the apex region 152 can form an angle 180 between the two angled struts 130 or 136 extending from either side of the corresponding apex region 152 (FIG. 3). In some instances, the angle 180 can be in a range of 120 (not inclusive) to 140 degrees (e.g., such that the angle 180 is greater than 120 degrees and less than or equal to 140 degrees).

Additional details and examples of frames for prosthetic heart valves that include apex regions can be found in U.S. Provisional Patent Application Nos. 63/178,416, filed April 22, 2021, 63/194,830, filed May 28, 2021, and 63/279,096, filed November 13, 2021.

FIG. 4 shows a delivery apparatus 200, according to an example, that can be used to implant an expandable prosthetic heart valve (e.g., the prosthetic heart valve 100 of FIG. 1 and/or any of the other prosthetic heart valves described herein). In some examples, the delivery apparatus 200 is specifically adapted for use in introducing a prosthetic valve into a heart.

The delivery apparatus 200 in the illustrated example of FIG. 4 is a balloon catheter comprising a handle 202 and a steerable, outer shaft 204 extending distally from the handle 202. The delivery apparatus 200 can further comprise an intermediate shaft 206 (which also may be referred to as a balloon shaft) that extends proximally from the handle 202 and distally from the handle 202, the portion extending distally from the handle 202 also extending coaxially through the outer shaft 204. Additionally, the delivery apparatus 200 can further comprise an inner shaft 208 extending distally from the handle 202 coaxially through the intermediate shaft 206 and the outer shaft 204 and proximally from the handle 202 coaxially through the intermediate shaft 206.

The outer shaft 204 and the intermediate shaft 206 can be configured to translate (e.g., move) longitudinally, along a central longitudinal axis 220 of the delivery apparatus 200, relative to one another to facilitate delivery and positioning of a prosthetic valve at an implantation site in a patient's body.

The intermediate shaft 206 can include a proximal end portion 210 that extends proximally from a proximal end of the handle 202, to an adaptor 212. A rotatable knob 214 can be mounted on the proximal end portion 210 and can be configured to rotate the intermediate shaft 206 around the central longitudinal axis 220 and relative to the outer shaft 204.

The adaptor 212 can include a first port 238 configured to receive a guidewire therethrough and a second port 240 configured to receive fluid (e.g., inflation fluid) from a fluid source. The second port 240 can be fluidly coupled to an inner lumen of the intermediate shaft 206.

The intermediate shaft 206 can further include a distal end portion that extends distally beyond a distal end of the outer shaft 204 when a distal end of the outer shaft 204 is positioned away from an inflatable balloon 218 of the delivery apparatus 200. A distal end portion of the inner shaft 208 can extend distally beyond the distal end portion of the intermediate shaft 206.

The balloon 218 can be coupled to the distal end portion of the intermediate shaft 206.

In some examples, a distal end of the balloon 218 can be coupled to a distal end of the delivery apparatus 200, such as to a nose cone 222 (as shown in FIG. 4), or to an alternate component at the distal end of the delivery apparatus 200 (e.g., a distal shoulder). An intermediate portion of the balloon 218 can overlay a valve mounting portion 224 of a distal end portion of the delivery apparatus 200 and a distal end portion of the balloon 218 can overly a distal shoulder 226 of the delivery apparatus 200. The valve mounting portion 224 and the intermediate portion of the balloon 218 can be configured to receive a prosthetic heart valve in a radially compressed state. For example, as shown schematically in FIG. 4, a prosthetic heart valve 250 (which can be one of the prosthetic valves described herein) can be mounted around the balloon 218, at the valve mounting portion 224 of the delivery apparatus 200.

The balloon shoulder assembly, including the distal shoulder 226, is configured to maintain the prosthetic heart valve 250 (or other medical device) at a fixed position on the balloon 218 during delivery through the patient's vasculature.

The outer shaft 204 can include a distal tip portion 228 mounted on its distal end. The outer shaft 204 and the intermediate shaft 206 can be translated axially relative to one another to position the distal tip portion 228 adjacent to a proximal end of the valve mounting portion 224, when the prosthetic valve 250 is mounted in the radially compressed state on the valve mounting portion 224 (as shown in FIG. 4) and during delivery of the prosthetic valve to the target implantation site. As such, the distal tip portion 228 can be configured to resist movement of the prosthetic valve 250 relative to the balloon 218 proximally, in the axial direction, relative to the balloon 218, when the distal tip portion 228 is arranged adjacent to a proximal side of the valve mounting portion 224.

An annular space can be defined between an outer surface of the inner shaft 208 and an inner surface of the intermediate shaft 206 and can be configured to receive fluid from a fluid source via the second port 240 of the adaptor 212. The annular space can be fluidly coupled to a fluid passageway formed between the outer surface of the distal end portion of the inner shaft 208 and an inner surface of the balloon 218. As such, fluid from the fluid source can flow to the fluid passageway from the annular space to inflate the balloon 218 and radially expand and deploy the prosthetic valve 250.

An inner lumen of the inner shaft can be configured to receive a guidewire therethrough, for navigating the distal end portion of the delivery apparatus 200 to the target implantation site.

The handle 202 can include a steering mechanism configured to adjust the curvature of the distal end portion of the delivery apparatus 200. In the illustrated example, for example, the handle 202 includes an adjustment member, such as the illustrated rotatable knob 260, which in turn is operatively coupled to the proximal end portion of a pull wire. The pull wire can extend distally from the handle 202 through the outer shaft 204 and has a distal end portion affixed to the outer shaft 204 at or near the distal end of the outer shaft 204. Rotating the knob 260 can increase or decrease the tension in the pull wire, thereby adjusting the curvature of the distal end portion of the delivery apparatus 200. Further details on steering or flex mechanisms for the delivery apparatus can be found in U.S. Patent No. 9,339,384.

The handle 202 can further include an adjustment mechanism 261 including an adjustment member, such as the illustrated rotatable knob 262, and an associated locking mechanism including another adjustment member, configured as a rotatable knob 278. The adjustment mechanism 261 is configured to adjust the axial position of the intermediate shaft 206 relative to the outer shaft 204 (e.g., for fine positioning at the implantation site). Further details on the delivery apparatus 200 can be found in PCT Application No. PCT/US2021/047056.

As introduced above, commissures formed with a leaflet assembly can extend through commissure windows in a frame of the prosthetic heart valve, thereby resulting in a portion of the commissures extending radially outward from an outer surface of the frame (e.g., as shown in FIG. 1 for commissures 114). The inventors herein have recognized that this can form step-like, protruding features on an exterior of the radially compressed prosthetic heart valve that can contact an inner wall of a delivery sheath through which a delivery apparatus is navigated en route to an implantation site. For example, the delivery sheath can be inserted into a vessel of a patient and extend into a patient's heart toward the implantation site and then the delivery apparatus, around which the prosthetic heart valve is mounted in the radially compressed configuration, can be navigated through an interior of the delivery sheath to the implantation site. As the delivery apparatus is moved through the delivery sheath, the protruding commissures may contact the inner wall of the delivery sheath, thereby causing the commissures to fold backward as the radially compressed prosthetic heart valve is pushed through the delivery sheath. As a result, a push force felt by a user navigating the delivery apparatus through the delivery sheath can be higher than desired.

In some examples, an outer skirt for a prosthetic heart valve (e.g., prosthetic heart valve 100) includes one or more axially extending (or vertical) extensions or extension portions that are configured to extend over and cover a portion of the commissures of the prosthetic heart valve. The extension portions of the outer skirt can be configured to slide more easily (e.g., with reduced friction) against a delivery sheath and/or prevent the commissures from backfolding as the radially compressed prosthetic heart valve is advanced through the delivery sheath toward an implantation site with a delivery apparatus. As a result, push forces felt by a user advancing the delivery apparatus through the delivery sheath can be reduced.

FIG. 5 shows one example of an outer skirt 300 for a prosthetic device, such as the prosthetic heart valve 100 of FIG. 1, in a flattened configuration. As described in further detail below, the outer skirt 300 comprises one or more extension portions (three shown in FIG. 5) extending outward from an outflow edge portion of the outer skirt 300 that are configured to cover the commissures of the prosthetic heart valve at least partially. The outer skirt 300 can be used in a mechanically expandable prosthetic valve, a balloon-expandable prosthetic valve (e.g., prosthetic heart valve 100 of FIG. 1), and/or a self-expandable prosthetic valve. Additional details on balloon expandable prosthetic valves can be found in U.S. Patent No. 9,393,110, and U.S. Provisional Application Nos. 63/178,416, filed April 22, 2021, 63/194,830, filed May 28, 2021, and 63/279,096, filed November 13, 2021. Additional details on a mechanically expandable prosthetic valve can be found in International Application PCT/US2021/052745, filed September 30, 2021. Additional details on a self-expanding prosthetic valve can be found in U.S. Patent No. 8,652,202.

The outer skirt 300 can be wrapped around and mounted to an outer surface of a frame of a prosthetic device (a radially outward facing surface relative to a central longitudinal axis of the prosthetic device), thereby transitioning to an annular configuration (e.g., as shown in FIG. 7). As an example, the outer skirt 300 is shown disposed around and secured to the outer surface of the frame 102 (FIGS. 7-11).

As shown in FIG. 5, the outer skirt 300 can comprise opposing first and second edge portions 302, 304 (which can also be referred to as short edges or edge portions) which each extend between an outflow edge portion 306 and an inflow edge portion 308 of the outer skirt 300. In some instances, the first and second edge portions 302, 304 can be non-parallel to a central longitudinal axis of the frame of the prosthetic device (when mounted around the frame) and/or non-perpendicular to the outflow edge portion 306. For example, the first and second edge portions 302, 304 can extend at angles of about 45 degrees (or in a range of 40 to 50 degrees) relative to the outflow edge portion 306 and/or a line extending in a circumferential direction of the frame. Therefore, an overall general shape of the outer skirt 300 can be that of a rhomboid or parallelogram.

In some examples, the first and second edge portions 302, 304 can each comprise a plurality of apertures 310 extending therethrough (e.g., the apertures spaced apart and extending in a line along the first and second edge portions 302, 304). Thus, when the outer skirt 300 is converted into its annular configuration (e.g., when mounted around a frame of a prosthetic device, as shown in FIG. 7 for example), the first and second edge portions 302, 304 can overlap one another with their respective apertures 310 overlapping as well. A suture can then be used to form a plurality of stitches in and in-and-out pattern through the overlapping apertures 310, thereby securing the first and second edge portions 302, 304 together and forming the annular configuration of the outer skirt 300.

As shown in FIG. 5 and the detail view of a portion of the outer skirt 300 in FIG. 6, the outer skirt 300 includes one or more extension portions 312 (or flaps) that extend outward (in an axial direction relative to a central longitudinal axis of a frame to which the outer skirt 300 is attached) from the outflow edge portion 306 (FIGS. 5 and 6). In some instances, the outer skirt 300 can include a plurality of extension portions 312 (e.g., one for each commissure window of the frame to which the outer skirt 300 is attached) that are spaced apart from one another along the outflow edge portion 306. A spacing between adjacent extension portions 312 can be selected such that each extension portion matches up with a commissure window 142 and corresponding commissure of the prosthetic heart valve when attached to the frame of the prosthetic heart valve (e.g., as shown in FIG. 7).

Each extension portion 312 can have a width 314 (in a circumferential direction) and axial length 316 (FIG. 6) that are sized to cover at least an inflow portion of a commissure 114 that extends through and exterior to a commissure window 142 of the frame 102, as shown in the schematic cross-sectional view of FIG. 9. In some examples, the width 314 can be specified such that it covers a width of the commissure (and the commissure tabs 115 forming the commissure 114) that extend outward from the commissure window 142 (the commissure tabs 115 of the commissure 114 shown in FIGS. 1 and 9), or another commissure support or feature of the frame to which the commissure is attached. As such, the extension portions 312 can be dimensioned such that they extend over at least an inflow portion of a respective commissure window 142 and the corresponding window struts 138 (or alternate commissure support or feature of the frame), as depicted in FIG. 7.

In some examples, the length 316 can be specified such that it covers an inflow portion or lower half of the commissure 114 (as shown in FIG. 9) and/or the commissure window 142 (as shown in FIG. 7). In alternate examples, the length 316 can be longer than shown in FIGS. 7 and 9 and cover a larger portion or an entirety of the commissure 114 and/or commissure window 142, thereby covering a larger portion (e.g., more than half) of the commissure 114 extending radially outward from the commissure window 142. In still other examples, the length 316 can be specified such that is covers 25-75% or 40-60% of a length of the commissure 114.

In some examples, the outflow edge portion 306 can be relatively straight (as shown in FIGS. 5-7). However, in alternate examples, the outflow edge portion 306 can have an undulating shape, such as shown in the provisional patent application under Edwards attorney docket No. 12131US01, as already referenced above. In such examples, the extension portions 312 can extend from peaks of the undulating outflow edge portion.

As shown in FIG. 9, each extension portion 312 covering a corresponding commissure 114 can form a gradually tapering covering over the inflow portion of the commissure 114 (e.g., tapering in a radial direction of the frame 102). For example, when radially compressed around a distal end portion of a delivery apparatus (e.g., the delivery apparatus 200 of FIG. 4), the inflow end 108 of the frame can be arranged closest to the distal (or leading) end of the delivery apparatus. As such, the direction of sliding or friction forces from a delivery sheath on the prosthetic heart valve, as the delivery apparatus and the radially compressed prosthetic heart valve are advanced through the delivery sheath, can be from the inflow end 108 toward the outflow end 110, as denoted by arrow 318 in FIG. 9. By providing the gradual increase in width or thickness in the radial direction from a first portion 320 of the outer skirt 300 covering an intermediate portion of the frame 102 to the extension portions 312 covering the radially protruding commissures 114, the radially compressed prosthetic heart valve can be more easily advanced through the delivery sheath en route to the implantation site. For example, the extension portions 312 can prevent the commissures 114 from back-folding under pressure from the delivery sheath. As a result, push forces felt by a user advancing the delivery apparatus through the delivery sheath can be reduced.

The extension portions 312 can be secured to their respective commissures 114 via one or more fasteners, such as one or more sutures 360 (FIG. 9). In some examples, as shown in FIG. 6, each extension portion 312 can comprise one or more apertures 362 through a material of the extension portion 312 that are configured to receive the suture 360 (or alternate fastener) and enable to the extension portion 312 to be more easily secured to the commissure 114. As shown in FIG. 6, the extension portions 312 each include two apertures 362. However, in alternate examples, the extension portions 312 can include more or less than two apertures 362 (e.g., one, three, four, or the like), or the extension portions 312 may not include any apertures and instead may be secured to the commissures 114 by a needle puncturing and extending through the material of the outer skirt 300.

The outer skirt 300 can comprise various synthetic materials, including fabrics (e.g., polyethylene terephthalate (PET) fabric or ultra high molecular weight polyethylene (UHMWPE) fabric), polytetrafluoroethylene (PTFE), thermoplastic polyurethane (TPU), a hybrid material comprising one or more fabric or polymeric materials (e.g., PET coated in TPU), or natural tissue (e.g., pericardial tissue). In some examples, the material of the outer skirt 300 can be selected to further reduce friction between the outer skirt 300 and the inner wall of the delivery sheath during advancement of the radially compressed prosthetic heart valve through the delivery sheath.

In some examples, the outer skirt 300 comprises two circumferentially extending stitch lines (or suture lines) proximate to the inflow edge portion 308, including a first stitch line 322 and a second stitch line 324 (FIG. 6). The first stitch line 322 comprises a plurality of in-and-out stitches 326 extending through and circumferentially along the outer skirt 300, from the first edge portion 302 to the second edge portion 304, at an axial location that is disposed proximate to but spaced away from the inflow edge portion 308. The second stitch line 324 comprises a plurality of in-and-out stitches 328 extending through and circumferentially along the outer skirt 300, from the first edge portion 302 to the second edge portion 304, at an axial location that is disposed adjacent to, but spaced away from, the first stitch line 322 and adjacent to the inflow edge portion 308. For example, as shown in FIG. 6, the second stitch line 324 is disposed between the inflow edge portion 308 and the first stitch line 322, with a gap 330 separating the first stitch line 322 and the second stitch line 324.

In some examples, the first stitch line 322 and the second stitch line 324 can be parallel to one another. In alternate examples, the first stitch line 322 and the second stitch line 324 can be non-parallel with one another. For examples, in some instances, the gap 330 can be constant between the first stitch line 322 and the second stitch line 324 circumferentially along the outer skirt 300. In alternate instances, the gap 330 can vary along the outer skirt 300, such as being larger at locations that align with the apex regions 152 at the inflow end 108 of the frame 102 (when the outer skirt 300 is mounted around the frame 102, as shown in FIG. 7). In such instances, a pocket of excess skirt material can be formed between the first stitch line 322 and the second stitch line 324 at the apex regions 152, which can facilitate elongation of the prosthetic heart valve during radially compressing the prosthetic heart valve (e.g., when crimping it onto the delivery apparatus).

In some instances, as shown in FIGS. 5 and 6, the stitches 326 of the first stitch line 322 can extend through pre-formed apertures 332 (or perforations) in the outer skirt 300, from a first surface 342 (outer surface) to a second surface 344 (inner surface) of the outer skirt 300, and the stitches 328 of the second stitch line 324 can extend through pre-formed apertures 334 (or perforations) in the outer skirt 300. In alternate instances, the stitches 326 of the first stitch line 322 and the stitches 328 of the second stitch line 324 can extend through a material of the outer skirt 300 (thereby forming apertures, e.g., with a needle).

As shown in FIGS. 5 and 6, one or both of the first stitch line 322 and the second stitch line 324 can have an undulating shape that approximately tracks a shape of the inflow struts 168 of the frame 102 (as shown in FIG. 7).

When the outer skirt 300 is disposed around an outer surface of the frame 102, as shown in FIG. 7, an inflow portion of the outer skirt 300 that includes the second stitch line 324 and the inflow edge portion 308 can be wrapped around the inflow struts 168 (as shown in the schematic cross-sectional view of FIG. 10). In some examples, as show schematically in the detail cross-sectional view 364 of FIG. 11, the inflow portion of the outer skirt 300 is wrapped around the inflow struts 168 such that the in-and-out stitches 328 of the second stitch line 324 are disposed on an inside 338 (or interior) of the frame 102 (on both the first surface 342 and second surface 344 of the outer skirt 300) and the in-and-out-stitches 326 of the first stitch line 322 are disposed on an outside 340 (or exterior) of the frame 102 (on both the first surface 342 and second surface 344 of the outer skirt 300). In this configuration, and due to the gap 330, the first stitch line 322 and the second stitch line 324 can be aligned with one another in the radial direction (e.g., above the inflow strut 168 in FIG. 1). The first stitch line 322 and the second stitch line 324 can then be secured together around the inflow struts 168.

For example, in some instances, a suture 336 (or other fastener) can extend through the stitches 326 of the first stitch line 322 and the stitches 328 of the second stitch line 324 that are disposed on the second surface 344 (inner surface) of the outer skirt 300 (FIG. 11), thereby connecting the first stitch line 322 and the second stitch line 324 together around the inflow struts 168 and covering the inflow struts 168 with a material of the outer skirt 300. As shown in FIG. 11, the stitches 326 and 328 are secured together around an axially inward facing surface 366 of the inflow struts 168 and the outer skirt 300 can cover an axially outward facing surface 368, a radially outward facing surface 370, and a radially inward facing surface 372 of the inflow struts 168.

In alternate instances, a suture (or other fastener) can extend through the stitches 326 of the first stitch line 322 that are disposed on the second surface 344 of the outer skirt 300 and the stitches 328 of the second stitch line 324 that are disposed on the first surface 342 of the outer skirt 300.

In some examples, the outer skirt 300 can also include a third stitch line 346 which follows a cusp edge of the leaflets when the leaflets and the outer skirt 300 are secured to the frame 102. Thus, the third stitch line 346 can also be referred to as a scallop stitch line. For example, as shown in FIG. 6, the third stitch line 346 can comprise a plurality of in-and-out stitches 348 that forming an undulating pattern between the outflow edge portion 306 and the first stitch line 322 of the outer skirt 300. As shown in FIGS. 6 and 7, the third stitch line 346 can have peaks adjacent to the extension portions 312 (and the commissure windows 142) and valleys at the first stitch line 322 (and thus, in some instances, the third stitch line 346 and the first stitch line 322 can overlap, intersect, or be formed from the same stitches proximate to the inflow edge portion 308).

In some instances, as shown in FIGS. 5 and 6, the stitches 348 of the third stitch line 346 can extend through pre-formed apertures 350 (or perforations) in the outer skirt 300. In alternate instances, the stitches 348 of the third stitch line 346 can extend through a material of the outer skirt 300 (thereby forming apertures, e.g., with a needle).

As shown in FIGS. 6 and 7, in some examples, the outer skirt 300 can also include a fourth stitch line 352 which undulates along an outflow portion of the outer skirt 300 (adjacent to the outflow edge portion 306) such that the fourth stitch line 352 extends along the angled struts 134 of the third row of angled struts (the angled struts connected to the axial struts 140 and window struts 138) when the outer skirt 300 is disposed around the frame 102 (FIG. 7). The fourth stitch line 352 can comprise a plurality of in-and-out stitches 354. In some instances, a portion of the fourth stitch line 352 can overlap, intersect, or be formed from the same stitches as the third stitch line 346 (e.g., along the angled struts 134 extending along the cusp edge or scallop line of the leaflets).

In some instances, as shown in FIGS. 5 and 6, the fourth stitch line 352 can extend through pre-formed apertures 356 (or perforations) in the outer skirt 300. In alternate instances, the stitches 254 of the fourth stitch line 352 can extend through a material of the outer skirt 300 (thereby forming apertures, e.g., with a needle).

Fasteners, such as whip stitches 358 can be used to secure the outer skirt 300 to the struts of the frame 102 via the stitches of the third stitch line 346 and the fourth stitch line 352 (FIG. 7). For example, as shown in FIG. 7, the whip stitches 358 can extend around the angled struts of the frame 102 and around the stitches 348 and 354 (between the stitches and a material of the outer skirt 300) that extend along the second surface 344 of the outer skirt 300 (which can be an inner surface of the outer skirt 300 that is disposed against an outer surface of the struts of the frame). **In** this way, the outer skirt 300 can be more easily secured to the frame 102 without extending the whip stitches 358 through a material of the outer skirt 300.

### Delivery Techniques

For implanting a prosthetic valve within the native aortic valve via a transfemoral delivery approach, the prosthetic valve is mounted in a radially compressed state along the distal end portion of a delivery apparatus. The prosthetic valve and the distal end portion of the delivery apparatus are inserted into a femoral artery and are advanced into and through the descending aorta, around the aortic arch, and through the ascending aorta. The prosthetic valve is positioned within the native aortic valve and radially expanded (e.g., by inflating a balloon, actuating one or more actuators of the delivery apparatus, or deploying the prosthetic valve from a sheath to allow the prosthetic valve to self-expand). Alternatively, a prosthetic valve can be implanted within the native aortic valve in a transapical procedure, whereby the prosthetic valve (on the distal end portion of the delivery apparatus) is introduced into the left ventricle through a surgical opening in the chest and the apex of the heart and the prosthetic valve is positioned within the native aortic valve. Alternatively, in a transaortic procedure, a prosthetic valve (on the distal end portion of the delivery apparatus) are introduced into the aorta through a surgical incision in the ascending aorta, such as through a partial **J-**sternotomy or right parasternal mini-thoracotomy, and then advanced through the ascending aorta toward the native aortic valve.

For implanting a prosthetic valve within the native mitral valve via a transseptal delivery approach, the prosthetic valve is mounted in a radially compressed state along the distal end portion of a delivery apparatus. The prosthetic valve and the distal end portion of the delivery apparatus are inserted into a femoral vein and are advanced into and through the inferior vena cava, into the right atrium, across the atrial septum (through a puncture made in the atrial septum), into the left atrium, and toward the native mitral valve. Alternatively, a prosthetic valve can be implanted within the native mitral valve in a transapical procedure, whereby the prosthetic valve (on the distal end portion of the delivery apparatus) is introduced into the left ventricle through a surgical opening in the chest and the apex of the heart and the prosthetic valve is positioned within the native mitral valve.

For implanting a prosthetic valve within the native tricuspid valve, the prosthetic valve is mounted in a radially compressed state along the distal end portion of a delivery apparatus. The prosthetic valve and the distal end portion of the delivery apparatus are inserted into a femoral vein and are advanced into and through the inferior vena cava, and into the right atrium, and the prosthetic valve is positioned within the native tricuspid valve. A similar approach can be used for implanting the prosthetic valve within the native pulmonary valve or the pulmonary artery, except that the prosthetic valve is advanced through the native tricuspid valve into the right ventricle and toward the pulmonary valve/pulmonary artery.

Another delivery approach is a transatrial approach whereby a prosthetic valve (on the distal end portion of the delivery apparatus) is inserted through an incision in the chest and an incision made through an atrial wall (of the right or left atrium) for accessing any of the native heart valves. Atrial delivery can also be made intravascularly, such as from a pulmonary vein. Still another delivery approach is a transventricular approach whereby a prosthetic valve (on the distal end portion of the delivery apparatus) is inserted through an incision in the chest and an incision made through the wall of the right ventricle (typically at or near the base of the heart) for implanting the prosthetic valve within the native tricuspid valve, the native pulmonary valve, or the pulmonary artery.

**In** all delivery approaches, the delivery apparatus can be advanced over a guidewire previously inserted into a patient's vasculature. Moreover, the disclosed delivery approaches are not intended to be limited. Any of the prosthetic valves disclosed herein can be implanted using any of various delivery procedures and delivery devices known in the art.

Any of the systems, devices, apparatuses, etc. herein can be sterilized (for example, with heat/thermal, pressure, steam, radiation, and/or chemicals, etc.) to ensure they are safe for use with patients, and any of the methods herein can include sterilization of the associated system, device, apparatus, etc. as one of the steps of the method. Examples of heat/thermal sterilization include steam sterilization and autoclaving. Examples of radiation for use in sterilization include, without limitation, gamma radiation, ultra-violet radiation, and electron beam. Examples of chemicals for use in sterilization include, without limitation, ethylene oxide, hydrogen peroxide, peracetic acid, formaldehyde, and glutaraldehyde. Sterilization with hydrogen peroxide may be accomplished using hydrogen peroxide plasma, for example.

The features described herein with regard to any example can be combined with other features described in any one or more of the other examples, unless otherwise stated. For example, any one or more of the features of one frame can be combined with any one or more features of another frame. As another example, any one or more features of one skirt can be combined with any one or more features of another skirt.

In view of the many possible ways in which the principles of the disclosure may be applied, it should be recognized that the illustrated configurations depict examples of the disclosed technology and should not be taken as limiting the scope of the disclosure nor the claims. Rather, the scope of the claimed subject matter is defined by the following claims.

## Claims

1. A prosthetic heart valve (100) comprising:
an annular frame (102) comprising a plurality of commissure features (138, 142);
a plurality of leaflets (112) arranged within an interior of the frame (102) and secured to one another at adjacent ends to form commissures (114), wherein each commissure (114) is connected to a respective commissure feature (138, 142) of the frame (102); and
an outer skirt (300) disposed around an outer surface of the frame (102), the outer skirt (300) comprising:
a first edge portion (308);
a second edge portion (306) secured to an intermediate portion of the frame (102) disposed between a first end and a second end of the frame (102); and
a plurality of extension portions (312) extending axially from the second edge portion (306), each extension portion (312) covering at least a portion of a corresponding commissure (114) on an exterior of the frame (102).

2. The prosthetic heart valve (100) of claim 1, wherein each extension portion (312) is formed as a flap that has a width (314) and length (316) sized to cover a width and at least a portion of a length of the corresponding commissure (114).

3. The prosthetic heart valve (100) of either claim 1 or claim 2, wherein each commissure (114) protrudes radially outward from the respective commissure feature (138, 142) and the outer surface of the frame (102) such that each extension portion (312) covering the corresponding commissure (114) forms an outer covering for the frame (102) that gradually tapers in a radial direction from the extension portion (312) covering the commissure (114) to the second edge portion (306) of the outer skirt (300).

4. The prosthetic heart valve (100) of any one of claims 1 to 3, wherein the prosthetic heart valve (100) includes three commissures (114) and three extension portions (312), wherein the first edge portion (308) is secured to the first end of the frame (102), and wherein the first end is an inflow end (108) of the frame (102), and wherein the second end is an outflow end (110) of the frame (102).

5. The prosthetic heart valve (100) of any one of claims 1 to 4, wherein the annular frame (102) comprises a plurality of interconnected struts, wherein the plurality of commissure features (138, 142) is a plurality of axially extending window struts (138) spaced apart around the frame (102), each axially extending window strut (138) defining a commissure window (142), and wherein each commissure (114) extends through and is secured to the commissure window (142) of a respective axially extending window strut (138).

6. The prosthetic heart valve (100) of claim 5, wherein the plurality of interconnected struts includes a circumferentially extending row of first struts defining the second end of the frame (102), a circumferentially extending row of second struts, and a plurality of axial struts (140), and wherein the plurality of axially extending window struts (138) and the plurality of axial struts (140) extend between the row of first struts and the row of second struts.

7. The prosthetic heart valve (100) of claim 6, wherein the second edge portion (306) is secured to first ends of the axial struts (140) that are connected to the row of second struts.

8. The prosthetic heart valve (100) of either claim 6 or claim 7, wherein the plurality of interconnected struts includes a circumferentially extending row of third struts (168) defining the first end of the frame (102), and wherein the first edge portion (308) is wrapped around and disposed on an inner surface of the third struts (168).

9. The prosthetic heart valve (100) of claim 8, wherein the outer skirt (300) includes a circumferentially extending first stitch line (322) comprising a plurality of in-and-out stitches (326, 328) and a circumferentially extending second stitch line (324) comprising a plurality of in-and-out stitches (326, 328), the first stitch line (322) and the second stitch line (324) spaced axially apart from one another in a flattened configuration of the outer skirt (300), and wherein the second stitch line (324) is disposed adjacent to the first edge portion (308).

10. The prosthetic heart valve (100) of claim 9, wherein the first stitch line (322) and the second stitch line (324) are radially aligned with one another and disposed on opposite sides of the third struts (168) such that a material of the outer skirt (300) disposed between the first and second stitch lines (324) covers a radially inward facing surface (372), a radially outward facing surface (370), and an axially outward facing surface (368) of the third struts (168).

11. A prosthetic heart valve (100) comprising:
an annular frame (102) comprising a plurality of interconnected struts and having an inflow end (108) and an outflow end (110); and
an outer skirt (300) disposed around an outer surface of the frame (102), the outer skirt (300) comprising:
an inflow edge portion (308) disposed at the inflow end (108);
an outflow edge portion (306) disposed at an intermediate portion of the frame (102);
a plurality of extension portions (312) extending axially from the outflow edge portion (306) and spaced circumferentially apart from one another, wherein each extension portion (312) extends toward the outflow end (110) of the frame (102); and
a first stitch line (322) and a second stitch line (324) extending circumferentially along an inflow portion of the outer skirt (300) that includes the inflow edge portion (308), and wherein the first stitch line (322) and the second stitch line (324) are spaced axially apart from one another in a flattened configuration of the outer skirt (300), and each comprise a plurality of in-and-out stitches (326, 328);
wherein the inflow portion of the outer skirt (300) is wrapped around a circumferentially extending row of inflow struts defining the inflow end (108) of the frame (102) such that the first stitch line (322) is disposed on the outer surface of the frame (102) and the second stitch line (324) is disposed on an inner surface of the frame (102) with the in-and-out stitches (326, 328) of the first stitch line (322) and the second stitch line (324) radially aligned with one another across the inflow struts.

12. The prosthetic heart valve (100) of claim 11, wherein the plurality of interconnected struts defines a plurality of rows of cells (118) arranged between the inflow end (108) and the outflow end (110), wherein the plurality of interconnected struts comprises a plurality of axially extending window struts (138) forming a plurality of commissure windows (142) spaced apart around the frame (102), and wherein each extension portion (312) extends over an inflow portion of a corresponding commissure window (142) of the plurality of commissure windows (142).

13. The prosthetic heart valve (100) of either claim 11 or claim 12, wherein a portion of the plurality of in-and-out stitches (326, 328) of the first stitch line (322) and the second stitch line (324) that are disposed on an inner surface of the outer skirt (300) are secured together at axially inward facing surfaces of the inflow struts by a suture that wraps around the portion of the plurality of in-and-out stitches (326, 328).

14. The prosthetic heart valve (100) of any one of claims 1 to 13, wherein the frame (102) is made of a plastically-expandable material and is configured to be crimped to a radially compressed state on a delivery catheter and then expanded inside a patient by an inflatable balloon.

15. The prosthetic heart valve (100) of any one of claims 1 to 14, wherein the prosthetic heart valve (100) is adapted to be implanted in the native aortic annulus.

## Patentansprüche

1. Herzklappenprothese (100), umfassend:
einen ringförmigen Rahmen (102), der eine Mehrzahl von Kommissurenmerkmalen (138, 142) umfasst;
eine Mehrzahl von Segeln (112), die in einem Inneren des Rahmens (102) angeordnet und an benachbarten Enden aneinander befestigt sind zum Bilden von Kommissuren (114), wobei jede Kommissur (114) mit einem jeweiligen Kommissurenmerkmal (138, 142) des Rahmens (102) verbunden ist; und
eine äußere Manschette (300), die um eine Außenfläche des Rahmens (102) herum angeordnet ist, wobei die äußere Manschette (300) folgendes umfasst:
einen ersten Randabschnitt (308);
einen zweiten Randabschnitt (306), der an einem Mittelabschnitt des Rahmens (102) befestigt ist, der zwischen einem ersten Ende und einem zweiten Ende des Rahmens (102) angeordnet ist; und
eine Mehrzahl von Verlängerungsabschnitten (312), die sich axial von dem zweiten Randabschnitt (306) erstrecken, wobei jeder Verlängerungsabschnitt (312) mindestens einen Abschnitt einer entsprechenden Kommissur (114) an einer Außenseite des Rahmens (102) bedeckt.

2. Herzklappenprothese (100) nach Anspruch 1, wobei jeder Verlängerungsabschnitt (312) als eine Lasche ausgebildet ist, die eine Breite (314) und eine Länge (316) aufweist, die so bemessen sind, dass sie eine Breite und mindestens einen Abschnitt einer Länge der entsprechenden Kommissur (114) bedecken.

3. Herzklappenprothese (100) nach Anspruch 1 oder Anspruch 2, wobei jede Kommissur (114) von dem jeweiligen Kommissurenmerkmal (138, 142) und der Außenfläche des Rahmens (102) radial nach außen vorsteht, so dass jeder Verlängerungsabschnitt (312), der die entsprechende Kommissur (114) bedeckt, eine äußere Abdeckung für den Rahmen (102) bildet, die sich in einer radialen Richtung von dem die Kommissur (114) bedeckenden Verlängerungsabschnitt (312) zu dem zweiten Randabschnitt (306) der äußeren Manschette (300) allmählich verjüngt.

4. Herzklappenprothese (100) nach einem der Ansprüche 1 bis 3, wobei die Herzklappenprothese (100) drei Kommissuren (114) und drei Verlängerungsabschnitte (312) umfasst, wobei der erste Randabschnitt (308) an dem ersten Ende des Rahmens (102) befestigt ist, und wobei das erste Ende ein Einströmende (108) des Rahmens (102) ist, und wobei das zweite Ende ein Ausströmende (110) des Rahmens (102) ist.

5. Herzklappenprothese (100) nach einem der Ansprüche 1 bis 4, wobei der ringförmige Rahmen (102) eine Mehrzahl von miteinander verbundenen Streben umfasst, wobei die Mehrzahl von Kommissurenmerkmalen (138, 142) eine Mehrzahl von sich axial erstreckenden Fensterstreben (138) ist, die um den Rahmen (102) herum voneinander beabstandet sind, wobei jede sich axial erstreckende Fensterstrebe (138) ein Kommissurenfenster (142) definiert, und wobei sich jede Kommissur (114) durch das Kommissurenfenster (142) einer jeweiligen sich axial erstreckenden Fensterstrebe (138) erstreckt und daran befestigt ist.

6. Herzklappenprothese (100) nach Anspruch 5, wobei die Mehrzahl von miteinander verbundenen Streben eine sich in Umfangsrichtung erstreckende Reihe erster Streben, die das zweite Ende des Rahmens (102) definiert, eine sich in Umfangsrichtung erstreckende Reihe zweiter Streben und eine Mehrzahl von Axialstreben (140) umfasst, und wobei sich die Mehrzahl von sich axial erstreckenden Fensterstreben (138) und die Mehrzahl von Axialstreben (140) zwischen der Reihe erster Streben und der Reihe zweiter Streben erstrecken.

7. Herzklappenprothese (100) nach Anspruch 6, wobei der zweite Randabschnitt (306) an ersten Enden der Axialstreben (140) befestigt ist, die mit der Reihe zweiter Streben verbunden sind.

8. Herzklappenprothese (100) nach Anspruch 6 oder Anspruch 7, wobei die Mehrzahl von miteinander verbundenen Streben eine sich in Umfangsrichtung erstreckende Reihe dritter Streben (168) umfasst, die das erste Ende des Rahmens (102) definiert, und wobei der erste Randabschnitt (308) um eine Innenfläche der dritten Streben (168) herumgewickelt und auf dieser angeordnet ist.

9. Herzklappenprothese (100) nach Anspruch 8, wobei die äußere Manschette (300) eine sich in Umfangsrichtung erstreckende erste Nahtlinie (322), die eine Mehrzahl von Ein-und-Aus-Stichen (326, 328) umfasst, und eine sich in Umfangsrichtung erstreckende zweite Nahtlinie (324), die eine Mehrzahl von Ein-und-Aus-Stichen (326, 328) umfasst, umfasst, wobei die erste Nahtlinie (322) und die zweite Nahtlinie (324) in einer abgeflachten Konfiguration der äußeren Manschette (300) axial voneinander beabstandet sind, und wobei die zweite Nahtlinie (324) benachbart zu dem ersten Randabschnitt (308) angeordnet ist.

10. Herzklappenprothese (100) nach Anspruch 9, wobei die erste Nahtlinie (322) und die zweite Nahtlinie (324) radial zueinander ausgerichtet und auf gegenüberliegenden Seiten der dritten Streben (168) angeordnet sind, so dass ein Material der äußeren Manschette (300), das zwischen der ersten und der zweiten Nahtlinie (324) angeordnet ist, eine radial nach innen weisende Fläche (372), eine radial nach außen weisende Fläche (370) und eine axial nach außen weisende Fläche (368) der dritten Streben (168) bedeckt.

11. Herzklappenprothese (100), umfassend:
einen ringförmigen Rahmen (102), der eine Mehrzahl von miteinander verbundenen Streben umfasst und ein Einströmende (108) und ein Ausströmende (110) aufweist; und
eine äußere Manschette (300), die um eine Außenfläche des Rahmens (102) herum angeordnet ist, wobei die äußere Manschette (300) umfasst:
einen Einströmrandabschnitt (308), der an dem Einströmende (108) angeordnet ist;
einen Ausströmrandabschnitt (306), der an einem Mittelabschnitt des Rahmens (102) angeordnet ist;
eine Mehrzahl von Verlängerungsabschnitten (312), die sich axial von dem Ausströmrandabschnitt (306) erstrecken und in Umfangsrichtung voneinander beabstandet sind, wobei sich jeder Verlängerungsabschnitt (312) zu dem Ausströmende (110) des Rahmens (102) hin erstreckt; und
eine erste Nahtlinie (322) und eine zweite Nahtlinie (324), die sich in Umfangsrichtung entlang eines Einströmabschnitts der äußeren Manschette (300) erstrecken, der den Einströmrandabschnitt (308) umfasst, und wobei die erste Nahtlinie (322) und die zweite Nahtlinie (324) in einer abgeflachten Konfiguration der äußeren Manschette (300) axial voneinander beabstandet sind und jeweils eine Mehrzahl von Ein-und-Aus-Stichen (326, 328) umfassen;
wobei der Einströmabschnitt der äußeren Manschette (300) um eine sich in Umfangsrichtung erstreckende Reihe von Einströmstreben herumgewickelt ist, die das Einströmende (108) des Rahmens (102) definiert, so dass die erste Nahtlinie (322) auf der Außenfläche des Rahmens (102) angeordnet ist und die zweite Nahtlinie (324) auf einer Innenfläche des Rahmens (102) angeordnet ist, wobei die Ein-und-Aus-Stiche (326, 328) der ersten Nahtlinie (322) und der zweiten Nahtlinie (324) über die Einströmstreben hinweg radial zueinander ausgerichtet sind.

12. Herzklappenprothese (100) nach Anspruch 11, wobei die Mehrzahl von miteinander verbundenen Streben eine Mehrzahl von Reihen von Zellen (118) definiert, die zwischen dem Einströmende (108) und dem Ausströmende (110) angeordnet sind, wobei die Mehrzahl von miteinander verbundenen Streben eine Mehrzahl von sich axial erstreckenden Fensterstreben (138) umfasst, die eine Mehrzahl von Kommissurenfenstern (142) bilden, die um den Rahmen (102) herum voneinander beabstandet sind, und wobei sich jeder Verlängerungsabschnitt (312) über einen Einströmabschnitt eines entsprechenden Kommissurenfensters (142) der Mehrzahl von Kommissurenfenstern (142) erstreckt.

13. Herzklappenprothese (100) nach Anspruch 11 oder Anspruch 12, wobei ein Teil der Mehrzahl von Ein-und-Aus-Stichen (326, 328) der ersten Nahtlinie (322) und der zweiten Nahtlinie (324), die auf einer Innenfläche der äußeren Manschette (300) angeordnet sind, an axial nach innen weisenden Flächen der Einströmstreben durch eine Naht aneinander befestigt sind, die um den Teil der Mehrzahl von Ein-und-Aus-Stichen (326, 328) herumgewickelt ist.

14. Herzklappenprothese (100) nach einem der Ansprüche 1 bis 13, wobei der Rahmen (102) aus einem plastisch expandierbaren Material hergestellt ist und dazu konfiguriert ist, auf einem Einführkatheter in einen radial komprimierten Zustand gecrimpt und dann in einem Patienten durch einen aufblasbaren Ballon expandiert zu werden.

15. Herzklappenprothese (100) nach einem der Ansprüche 1 bis 14, wobei die Herzklappenprothese (100) dazu angepasst ist, in den nativen Aortenanulus implantiert zu werden.

## Revendications

1. Valve cardiaque prothétique (100) comprenant :
un cadre annulaire (102) comprenant une pluralité d'éléments de commissure (138, 142) ;
une pluralité de feuillets (112) agencés à l'intérieur du cadre (102) et fixés les uns aux autres au niveau d'extrémités adjacentes pour former des commissures (114), chaque commissure (114) étant reliée à un élément de commissure (138, 142) respectif du cadre (102) ; et
une jupe externe (300) disposée autour d'une surface externe du cadre (102), la jupe externe (300) comprenant :
une première partie de bord (308) ;
une seconde partie de bord (306) fixée à une partie intermédiaire du cadre (102) disposée entre une première extrémité et une seconde extrémité du cadre (102) ; et
une pluralité de parties d'extension (312) s'étendant axialement depuis la seconde partie de bord (306), chaque partie d'extension (312) recouvrant au moins une partie d'une commissure (114) correspondante sur un extérieur du cadre (102).

2. Valve cardiaque prothétique (100) selon la revendication 1, dans laquelle chaque partie d'extension (312) est formée sous la forme d'un rabat qui présente une largeur (314) et une longueur (316) dimensionnées pour recouvrir une largeur et au moins une partie d'une longueur de la commissure (114) correspondante.

3. Valve cardiaque prothétique (100) selon l'une ou l'autre de la revendication 1 ou de la revendication 2, dans laquelle chaque commissure (114) fait saillie radialement vers l'extérieur depuis l'élément de commissure (138, 142) respectif et la surface externe du cadre (102) de telle sorte que chaque partie d'extension (312) recouvrant la commissure (114) correspondante forme un revêtement externe pour le cadre (102) qui s'effile progressivement dans une direction radiale depuis la partie d'extension (312) recouvrant la commissure (114) jusqu'à la seconde partie de bord (306) de la jupe externe (300).

4. Valve cardiaque prothétique (100) selon l'une quelconque des revendications 1 à 3, dans laquelle la valve cardiaque prothétique (100) comporte trois commissures (114) et trois parties d'extension (312), dans laquelle la première partie de bord (308) est fixée à la première extrémité du cadre (102), et dans laquelle la première extrémité est une extrémité d'entrée (108) du cadre (102), et dans laquelle la seconde extrémité est une extrémité de sortie (110) du cadre (102).

5. Valve cardiaque prothétique (100) selon l'une quelconque des revendications 1 à 4, dans laquelle le cadre annulaire (102) comprend une pluralité d'étais interconnectés, dans laquelle la pluralité d'éléments de commissure (138, 142) est une pluralité d'étais de fenêtre s'étendant axialement (138) espacés autour du cadre (102), chaque étai de fenêtre s'étendant axialement (138) définissant une fenêtre de commissure (142), et dans laquelle chaque commissure (114) s'étend à travers la fenêtre de commissure (142) d'un étai de fenêtre s'étendant axialement (138) respectif et est fixée à celle-ci.

6. Valve cardiaque prothétique (100) selon la revendication 5, dans laquelle la pluralité d'étais interconnectés comporte une rangée s'étendant circonférentiellement de premiers étais définissant la seconde extrémité du cadre (102), une rangée s'étendant circonférentiellement de deuxièmes étais, et une pluralité d'étais axiaux (140), et dans laquelle la pluralité d'étais de fenêtre s'étendant axialement (138) et la pluralité d'étais axiaux (140) s'étendent entre la rangée de premiers étais et la rangée de deuxièmes étais.

7. Valve cardiaque prothétique (100) selon la revendication 6, dans laquelle la seconde partie de bord (306) est fixée à des premières extrémités des étais axiaux (140) qui sont reliés à la rangée de deuxièmes étais.

8. Valve cardiaque prothétique (100) selon l'une ou l'autre de la revendication 6 ou de la revendication 7, dans laquelle la pluralité d'étais interconnectés comporte une rangée s'étendant circonférentiellement de troisièmes étais (168) définissant la première extrémité du cadre (102), et dans laquelle la première partie de bord (308) est enroulée autour d'une surface interne des troisièmes étais (168) et disposée sur celle-ci.

9. Valve cardiaque prothétique (100) selon la revendication 8, dans laquelle la jupe externe (300) comporte une première ligne de couture (322) s'étendant circonférentiellement comprenant une pluralité de points d'entrée et de sortie (326, 328) et une seconde ligne de couture (324) s'étendant circonférentiellement comprenant une pluralité de points d'entrée et de sortie (326, 328), la première ligne de couture (322) et la seconde ligne de couture (324) étant espacées axialement l'une de l'autre dans une configuration aplatie de la jupe externe (300), et dans laquelle la seconde ligne de couture (324) est disposée de manière adjacente à la première partie de bord (308).

10. Valve cardiaque prothétique (100) selon la revendication 9, dans laquelle la première ligne de couture (322) et la seconde ligne de couture (324) sont alignées radialement l'une avec l'autre et disposées sur des côtés opposés des troisièmes étais (168) de telle sorte qu'un matériau de la jupe externe (300) disposé entre les première et seconde lignes de couture (324) recouvre une surface tournée radialement vers l'intérieur (372), une surface tournée radialement vers l'extérieur (370) et une surface tournée axialement vers l'extérieur (368) des troisièmes étais (168).

11. Valve cardiaque prothétique (100) comprenant :
un cadre annulaire (102) comprenant une pluralité d'étais interconnectés et présentant une extrémité d'entrée (108) et une extrémité de sortie (110) ; et
une jupe externe (300) disposée autour d'une surface externe du cadre (102), la jupe externe (300) comprenant :
une partie de bord d'entrée (308) disposée au niveau de l'extrémité d'entrée (108) ;
une partie de bord de sortie (306) disposée au niveau d'une partie intermédiaire du cadre (102);
une pluralité de parties d'extension (312) s'étendant axialement depuis la partie de bord de sortie (306) et espacées circonférentiellement l'une de l'autre, chaque partie d'extension (312) s'étendant vers l'extrémité de sortie (110) du cadre (102); et
une première ligne de couture (322) et une seconde ligne de couture (324) s'étendant circonférentiellement le long d'une partie d'entrée de la jupe externe (300) qui comporte la partie de bord d'entrée (308), la première ligne de couture (322) et la seconde ligne de couture (324) étant espacées axialement l'une de l'autre dans une configuration aplatie de la jupe externe (300) et comprenant chacune une pluralité de points d'entrée et de sortie (326, 328) ;
dans laquelle la partie d'entrée de la jupe externe (300) est enroulée autour d'une rangée s'étendant circonférentiellement d'étais d'entrée définissant l'extrémité d'entrée (108) du cadre (102) de telle sorte que la première ligne de couture (322) est disposée sur la surface externe du cadre (102) et la seconde ligne de couture (324) est disposée sur une surface interne du cadre (102), les points d'entrée et de sortie (326, 328) de la première ligne de couture (322) et de la seconde ligne de couture (324) étant alignés radialement les uns avec les autres à travers les étais d'entrée.

12. Valve cardiaque prothétique (100) selon la revendication 11, dans laquelle la pluralité d'étais interconnectés définit une pluralité de rangées de cellules (118) agencées entre l'extrémité d'entrée (108) et l'extrémité de sortie (110), dans laquelle la pluralité d'étais interconnectés comprend une pluralité d'étais de fenêtre s'étendant axialement (138) formant une pluralité de fenêtres de commissure (142) espacées autour du cadre (102), et dans laquelle chaque partie d'extension (312) s'étend sur une partie d'entrée d'une fenêtre de commissure (142) correspondante de la pluralité de fenêtres de commissure (142).

13. Valve cardiaque prothétique (100) selon l'une ou l'autre de la revendication 11 ou de la revendication 12, dans laquelle une partie de la pluralité de points d'entrée et de sortie (326, 328) de la première ligne de couture (322) et de la seconde ligne de couture (324) qui sont disposés sur une surface interne de la jupe externe (300) sont fixés ensemble au niveau de surfaces tournées axialement vers l'intérieur des étais d'entrée par une suture qui s'enroule autour de la partie de la pluralité de points d'entrée et de sortie (326, 328).

14. Valve cardiaque prothétique (100) selon l'une quelconque des revendications 1 à 13, dans laquelle le cadre (102) est réalisé en un matériau plastiquement expansible et est configuré pour être serti dans un état radialement comprimé sur un cathéter de pose puis expansé à l'intérieur d'un patient par un ballonnet gonflable.

15. Valve cardiaque prothétique (100) selon l'une quelconque des revendications 1 à 14, dans laquelle la valve cardiaque prothétique (100) est adaptée pour être implantée dans l'anneau aortique natif.
